# EUROPEAN PATENT SPECIFICATION

(11) **EP 3 317 786 B1**
(45) Date of publication and mention of the grant of the patent: **13.09.2023**
(21) Application number: 16734625.3
(22) Date of filing: 30.06.2016
(51) Int. Cl.: G16H 20/30, G16H 40/63

(54) **CARDIOPULMONARY RESUSCITATION COORDINATION METHOD, COMPUTER PROGRAM PRODUCT AND SYSTEM**
KOORDINATIONSVERFAHREN ZUR KARDIOPULMONALEN WIEDERBELEBUNG, COMPUTERPROGRAMMPRODUKT UND SYSTEM
PROCÉDÉ DE COORDINATION DE RÉANIMATION CARDIO-PULMONAIRE, PRODUIT LOGICIEL ET SYSTÈME

(30) Priority: 30.06.2015 EP 15174595
(43) Date of publication of application: 09.05.2018
(73) Proprietor: Koninklijke Philips N.V., 5656 AG Eindhoven (NL)
(72) Inventor: DELLIMORE, Kiran, Hamilton, J., 5656 AE Eindhoven (NL); MÜHLSTEFF, Jens, 5656 AE Eindhoven (NL)
(74) Representative: Philips Intellectual Property & Standards
(86) International application number: PCT/EP2016/065298
(87) International publication number: WO 2017/001555

(56) References cited:
- EP-A1- 2 851 831
- WO-A1-2013/009288
- US-A1- 2006 187 017
- US-A1- 2009 181 653
- US-A1- 2010 005 508

## Description

### FIELD OF THE INVENTION

The present invention relates to a cardiopulmonary resuscitation coordination method between a coordinator and a plurality of portable computing devices.

The present invention further relates to a computer program product for implementing such a cardiopulmonary resuscitation coordination method.

The present invention yet further relates to a cardiopulmonary resuscitation coordination system for executing the computer program product.

### BACKGROUND OF THE INVENTION

Worldwide a large number of people suffer sudden cardiac arrest (SCA) events. For instance, in Europe alone there are between 350,000 and 700,000 out of hospital sudden cardiac arrest (SCA) events every year which are frequently witnessed by (untrained) lay people. Cardiopulmonary resuscitation (CPR) is the key life-saving intervention recommended by international guidelines for victims of SCA, where necessary (in about 1/3 of all SCA cases) supplemented by defibrillation using an automatic external defibrillator (AED). CPR involves the delivery of chest compressions (CCs) at a depth of at least 5 cm and at a rate of 100 compressions per minute (cpm), in combination with artificial ventilation (i.e., rescue breaths) to maintain the circulatory flow and oxygen supply to the vital organs in the body of the SCA sufferer until spontaneous circulation returns.

For successful resuscitation it is essential that CPR is performed as soon as possible. Currently, international guidelines recommend that the cardiac arrest situation is recognised within 5-10 sec following the SCA event by performing a breathing check. Prior to 2005, a manual pulse check in addition to the breathing check was recommended by the guidelines for the diagnosis of cardiac arrest. This recommendation was removed from the CPR guidelines due to the unwillingness and the inability of lay rescuers to quickly and correctly perform pulse palpation. Nevertheless, pulse detection is still perceived by the resuscitation community as an important (supplementary) technique for the assessment of the need for CPR in an emergency situation.

With every minute that passes prior to initiating CPR, the probability of saving a victim's life decreases by 10% and after 10 minutes there is almost no chance of successful resuscitation.

Among the main barriers to bystander intervention when an SCA event occurs in a crowd, in a public setting, is that many individuals are uncertain whether to help since they expect that others will provide assistance and because there is often confusion over which person in the crowd should take charge of the resuscitation efforts. Hence, there is a profound need for a tool that can assist in the optimization of the CPR workflow by lay rescuer bystanders in pubic settings.

Hence, there is a profound need for a solution that can assist lay rescuers in the management of the CPR workflow of lay rescuer bystanders in pubic settings.

Many commercial solutions exist for CPR workflow management and CC guidance during CPR; however, these solutions are primarily aimed at meeting the needs of professional, Basic Life Support (BLS) and Advanced Life Support (ALS) users. In recent times some solutions aimed at lay rescuers have become available or are currently under development.

In addition, recently there has been a proliferation of smart device applications for lay rescuer CPR guidance, e.g., the Pocket First Aid & CPR, the Real time CPR guide and the CPR metronome, which can be obtained in app stores around the world, e.g. the Google Play Store. However, these apps are primarily for training and are unsuitable for real-time CPR coordination or guidance because they require continuous user input and interaction, again hampering CPR workflow.

Document US 2009/181653 A1 relates to a method in which mobile phone users are provided with the ability to discover personal attributes including photo of other individuals in vicinity covered by a short range wireless network. The users can elect to exchange, send or receive contact information with ones that are in the vicinity that includes photos. There are, however, not disclosed activity and role distribution and command reception in case of medical urgency situations. Further, no determination of a respective suitability indicator for each of the users associated with the identified portable computing devices is disclosed.

Document WO 2013/009288 A1 describes a method for trusted communication among mobile devices, in which information transmitted wirelessly from a first mobile device to a second mobile device permits the second mobile device to detect proximity of the first mobile device. In response to the information, the first mobile device receives a message and a first authentication value from the second mobile device, and then a second authentication value is generated at the first mobile device based on the message and a shared secret value. Integrity of the message can then be verified based on comparing the first authentication value and the second authentication value.

Finally, EP 2 851 831 A1 relates to a method for using a mobile information gateway, which includes a wearable human interface module, for home healthcare and that comprises capturing information with the wearable human interface module, processing the captured information to determine an identity of a user of the wearable human interface module, authenticating the identity of user, retrieving information using the captured information and the authenticated identity, and presenting the retrieved information using the wearable human interface module.

### SUMMARY OF THE INVENTION

The invention is solely defined in the appended claims.

The present invention seeks to provide a CPR coordination method that overcomes at least some of these drawbacks.

The present invention further seeks to provide a computer program product comprising a computer-readable medium embodying computer program code for implementing this method.

The present invention yet further seeks to provide a CPR coordination system for executing this computer program code.

According to an aspect, there is provided a cardiopulmonary resuscitation (CPR) coordination method according to claim 1.

Embodiments may provide CPR coordination guidance that accounts for available lay rescuers who positively or negatively influence the delivery of effective CPR, e.g., a highly skilled medical professional, an untrained individual, a first-aid-trained individual, a person with CPR experience, etc. Furthermore, embodiments may be portable and suitable for ubiquitous access during an SCA event or emergency situation, enabling to build a ubiquitous network from scratch to provide CPR coordination guidance in a collaborative manner.

Moreover, as there is a clear trend in society towards smart portable computing devices, it is reasonable to assume that a significant number of lay persons will be in the possession of a portable computing device, thereby increasing the likelihood of a SCA sufferer being provided with life-saving CPR, i.e. CPR that is administered safely and correctly due to the coordination guidance provided by the method of an embodiment.

In some embodiments, the coordinator may also be a portable computing device (e.g. a smartphone, a smartwatch or a Google glass) associated to a first user that recognizes the SCA event or emergency situation and initiates the method on said portable computing device.

The data acquired with the sensor component of a portable computing device assigned with a sensor functionality provides the coordinator with valuable information about the user of said portable computing device (e.g. vital signs information of said user may indicate that the user becomes tired after performing a CPR task for a while and should be replaced by another rescuer), information about the patient (e.g. vital signs information derived from the captured images, sounds or acceleration signals) and/or the area around the coordinator (e.g. assessment of potential hazards in the vicinity of the coordinator and the patient based on the recordings of the camera and/or microphone of the device).

The method advantageously identifies portable computing devices held by other users that are within a given distance range of the coordinator. The users of said identified devices may become potential collaborators to perform a CPR task. In some embodiments, the step of identifying a plurality of nearby portable computing devices comprises: determining their location by means of a respective GPS sensor incorporated in each of said devices and communicating their location via a telecommunication network or the cloud. In other embodiments, said step of identifying a plurality of nearby portable computing devices comprises the coordinator connecting locally with said devices via a short-range (e.g. 20-100 m) wireless connectivity network such as Wi-Fi, Bluetooth or Zigbee.

Preferably, the range of the area around the coordinator used for locating portable computing devices may be adjusted dynamically to increase or decrease the number of possible nearby devices with users who may be potential collaborators (e.g., if too many devices are found the range will be reduced, while if too few devices are located then the range will be increased).

The step of processing device-attribute information comprises, for each respective sensor functionality of said plurality of sensor functionalities, extracting a respective indicator of the sensor component of the identified portable computing devices from said device-attribute information, said respective indicator being indicative of the suitability of the sensor component of the identified portable computing devices for performing said respective sensor functionality.

The extracted indicators of the sensor components for a given sensor functionality may be used to rank or prioritize the sensor components in order of their suitability for said given sensor functionality. The respective sensor functionalities arethus be assigned to the sensor components of the identified portable computing devices based on said respective ranking or prioritization.

The device-attribute information may comprise at least one of: sensor components available on the portable computing device; battery specifications; model number information (e.g. device age); and supported wireless communication standards. Other device-attribute information may relate to the ability of the devices to receive data through a cellular or wireless network (e.g. roaming vs. non-roaming status, subscription vs. pre-paid plan, etc.).

The device-attribute information may, for example, be processed to assess and identify sensor components comprised in the portable computing devices identified in the vicinity of an SCA or accident victim which may be more suitable for performing certain sensor functionalities. The data acquired using these sensor components may allow to deliver more accurate cardiopulmonary resuscitation coordination guidance to perform more effective CPR.

Preferably, the step of processing user-attribute information may comprise: accessing a database of user-attribute information and extracting indicators for each of the users associated with the identified portable computing devices from said user-attribute information. For example, if user-attribute information is indicative that a user has little to no medical/first-aid knowledge or experience, an indicator may be extracted to indicate that the user is of low (or zero) suitability for performing cardiopulmonary resuscitation. The indicator may even be indicative that such a user (e.g. a lay rescuer) should not be permitted to intervene. In such an instance, the unsuitable user may be further guided to a safe nearby location to await the arrival of emergency services (e.g. using location information obtained by a GPS tracker of the system).

In an embodiment, the step of processing user-attribute information may further comprise: prioritizing the users based on the extracted indicators. For example, the extracted indicators for the users may be used to rank or prioritize the users in order of their suitability for CPR performance. One or more cardiopulmonary resuscitation roles may thus be assigned to the users based on such ranking or prioritization.

For example, prioritizing the users may employ a suitability index-based decision model. Such a decision model may use a state machine in order to determine decisions on prioritization.

The user-attribute information may comprise at least one of: medical qualification information; resuscitation experience information; medical training information; and physical condition information, for instance to assess if a certain individual is suitable or unsuitable for performing CPR. Also, the user-attribute information may enable the assessment and identification of individuals which could adversely affect the delivery of effective CPR.

The user-attribute information employed by an embodiment may therefore relate to medical training, medical qualification, or medical practice experience attributes of a user. However, it is to be understood that the user-attribute information may also relate to information that could, in theory, be considered to not relate to medical information in its strict or literal sense. For instance, user-attribute information employed by an embodiment may physical and/or mental attributes of user, and such information could potentially be thought of as not being 'medical information' in a strict sense of the expression. Accordingly, the skilled person would appreciate that user-attribute information employed by an embodiment may comprise many different types of information which may be useful for the purpose of assessing an individual's suitability and/or readiness for performing CPR.

The user-attribute information, for example, is processed to assess and identify medically/CPR trained persons in the vicinity of an SCA or accident victim who may be more suitable for performing CPR. By way of example, medically/CPR trained persons may be identified by referring to medical qualification information or by detecting an indicator, sign or signal that identifies a person as being qualified and/or experienced at CPR application. Determining an indicator of the suitability of a person for performing cardiopulmonary resuscitation on the patient may thus be accomplished via data, image and/or sound classification algorithms incorporating face, object and sound recognition. Such processing may be done on the `Cloud' (e.g. via a distributed processing environment).

Additionally, user-attribute information may be provided by other sources or services. For example, medical experience information, physical condition information from a database and/or sensors may be used.

An embodiment may further comprise: capturing user-attribute information from the sensor component of an identified portable computing device, wherein said sensor component optionally comprises at least one of: a sensor for monitoring vital signs of the user associated with the identified portable computing device; and a sensor adapted to capture an indicator in the form of a response of the user associated with the identified portable computing device to one or more questions presented to the user. Thus, an embodiment may be used to assess and account for a user's willingness or readiness to participate in CPR performance. This may be accomplished by implementing a heart rate and/or breathing classification algorithm which will analyze vital signs of the user, for example. Alternatively, the user's willingness or readiness can be assessed by prompting the user to provide a response to one or more questions (which may require an image and/or sound processing and classification algorithm to analyze the user response). Moreover, to increase the reliability of such an assessment using a portable computing device, both vital signs and user response(s) can be used together to confirm/qualify a user's indicated willingness or readiness.

In an embodiment, the step of communicating cardiopulmonary resuscitation coordination guidance may comprise: communicating first instructions for performing cardiopulmonary resuscitation via the communication link to a first portable computing device, the user associated to the first portable computing device being a primary user; and communicating second instructions for performing cardiopulmonary resuscitation via the communication link to one or more portable computing devices other than the first portable computing device, the respective user or users associated to said one or more user associated to said one or more portable computing devices being an assistant user or users for assisting to the primary user. For example, one user may be instructed to perform CPR on the victim while another user may be instructed to control bystanders (e.g. by directing then to a certain location away from the scene of the SCA or accident) and await the arrival of emergency services.

An embodiment may further comprise: the coordinator receiving sensor functionality response information from an identified portable computing device for indicating acceptance of a sensor functionality assigned to the sensor component of said identified device; processing said sensor functionality response information; and communicating adjusted cardiopulmonary resuscitation coordination guidance from the coordinator to at least one of the identified devices via the communication link in response to processed sensor functionality response information and the acquired data.

Preferably, such embodiment may further comprise the coordinator communicating to said identified portable computing device via the communication link sensor control commands for controlling the sensor component of said identified device.

An embodiment may further comprise: the coordinator receiving role response information from an identified portable computing device for indicating acceptance of an assigned cardiopulmonary resuscitation role in response to the cardiopulmonary resuscitation coordination guidance communicated to said identified device; processing said role response information; and communicating adjusted cardiopulmonary resuscitation coordination guidance from the coordinator to at least one of the identified devices via the communication link in response to processed role response information and the acquired data. For example, a user's reluctance to administer CPR (e.g. due to being physically tired, injured or disabled) may be indicated by the user by non-acceptance of an assigned role, and such non-acceptance can be accounted for by communicating adjusted CPR coordination guidance which reassigns one or more CPR role(s).

The portable computing devices may comprise a smartphone, smartwatch, personal digital assistant or tablet computer. Also, a portable computing device may be a head-mountable computing device having at least one display module arranged to be viewed by the wearer of the head-mountable computing device when wearing the device. Embodiments may thus employ conventional and widely-available portable computing devices that are commonly carried or worn by a significant number of people.

Embodiments may therefore utilize the insight that lay person rescuers or bystanders may carry (smart) portable computing devices that are capable of performing communicating information, such that a CPR guidance system may be compiled in situ by integrating devices in the vicinity of the rescuer into the CPR guidance system, e.g. in an automated or user-controller manner. The method may further comprise generating an alert via a user interface for alerting a user to enable a wireless communication mode of an identified device. In this manner, devices having the desired sensing capability can be manually configured such that they can be included into a CPR guidance system according to an embodiment.

Also, at least one of the identified portable computing devices may comprise a location sensor for detecting the location of the portable computing device. An embodiment may thus comprise sending a distress signal to a remote server (e.g. a server of an emergency service), said distress signal including location information (e.g. global positioning information) indicative of the detected location of the portable computing device. This may ensure that the victim will receive professional medical help as soon as possible.

Preferably, the sensor component of one or more of the plurality of portable computing devices may comprise at least one of: a camera; a microphone; an accelerometer; a location sensor; a plethysmography sensor; and a sensor for monitoring at least one vital sign. In this manner the data acquired with said sensor component will enable to perform hazard assessment of the environment in which the patient to be treated is located, video and/or audio documentation of the CPR, pulse check (e.g. by means of measuring the heart rate or the heart rate variability) and/or breathing check (e.g. by means of measuring the respiratory rate).

In an embodiment, the method may further comprise automatically identifying a defibrillator device in the vicinity of the patient; and communicating from the coordinator to said defibrillator device user instructions for said defibrillator device to be rendered to a user via a user interface of said defibrillator device. By detecting such a defibrillator device (and preferably checking its readiness or availability), defibrillation can be added to the CPR workflow, thus further improving the survival chances of the victim especially when defibrillation significantly improves such survival chances.

According to another aspect, there is provided a computer-readable medium embodying computer program code for implementing the method of any of the above embodiments when executed on a processor of a coordinator. Such a computer program product, when executed on a CPR guidance system of the present disclosure, facilitates the coordination and/or administration of particularly effective CPR as explained in more detail above.

According to yet another aspect, there is provided a cardiopulmonary resuscitation coordination system including the above computer program product, a coordinator, and a plurality of portable computing devices located within an area around the coordinator, said area comprising a patient to be treated, each of the plurality of portable computing devices being associated with a respective user and comprising a communication interface arranged to communicate information via a communication link, and a sensor component arranged to acquire data of the user of said portable computing device, the patient and/or the area around the coordinator. Such a CPR guidance system facilitates laypersons or bystanders to be presented with CPR coordination guidance that may assist lay rescuers in the assessment and management of a CPR administration during an SCA event or other emergency situation so as to enable them to deliver effective CPR in a timely manner. Moreover, by coordinating the sensor components of different portable computing devices, ubiquitous devices can be combined, e.g. in situ, to form a CPR guidance system according to an embodiment, thus increasing the chances of such a CPR guidance system being available in the event of an out of hospital SCA event.

The CPR guidance system may further comprise a database of user-attribute information, which may be integrated in one or the identified portable computing devices and/or remotely accessible via a communication network for example or via a distributed processing environment (such as the 'cloud' for example).

The CPR guidance system may be a modular system wherein the identified portable computing devices are separate modules, e.g. separate devices in communication with each other, e.g. via wireless communication.

Also, the sensor component of at least one of the plurality of portable computing devices may be adapted for capturing user-attribute information, wherein said sensor component optionally comprises at least one of: a sensor for monitoring vital signs of a user associated with the portable computing device; and a sensor adapted to capture an indicator in the form of a response of the user associated with the portable computing device to one or more questions presented to the user.

For example, each of the portable or wearable computing devices may comprise a smart phone, tablet, head-mountable computing device or smart watch.

### BRIEF DESCRIPTION OF THE DRAWINGS

Embodiments and illustrative examples of the disclosure are described in more detail and by way of non-limiting examples with reference to the accompanying drawings, wherein:
FIG. 1 schematically depicts an embodiment of a CPR coordination system of the disclosure;
FIG. 2 schematically depicts an aspect of the CPR coordination system of FIG. 1 in more detail;
FIG. 3 schematically depicts another embodiment of a CPR coordination system of the disclosure; and
FIG. 4 is a flowchart of an embodiment of a CPR coordination method of the disclosure.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

In the context of the present application, a portable computing device is a computing device that may be worn or carried by a person. An example of a portable computing device is a head-mountable computing device, which comprises a device that can be worn of the head of its user and provides the user with computing functionality. Non-limiting examples of such head-mountable computing devices include smart headgear, e.g. eyeglasses, goggles, a helmet, a hat, a visor, a headband, or any other device that can be supported on or from the wearer's head, and so on. Another example of a portable computing device is a smart watch, which is a device that can be worn on the wrist of it user and provide the user with computing functionality (in addition to the normal time-keeping functionality of a watch or time-piece). Further examples of portable computing devices include mobile phones, tablet computers, personal digital assistants, and laptop personal computers. The portable computing device may be configured to perform specific computing tasks as specified in a software application (app) that may be retrieved from the Internet or another computer-readable medium.

Embodiments may be arranged to use existing sensor components, processing capabilities, data storage, and/or communication capabilities of one or more portable computing devices (such as a smart watch, smartphone or other wearable sensor platform), to build a ubiquitous network of devices from scratch and, in this manner, aid lay rescuers in the coordination of multiple laypersons during an SCA event or emergency situation.

Moreover, conventional and widely-available smart devices typically incorporate many different sensors which are suitable for the capture or assessment of physical condition, location and user indications. These include a GPS tracker, a microphone, a camera, a heartbeat detector, or a sophisticated user interface (e.g., a LCD display, AMOLED, etc.) capable of visual and audible guidance, as well as a telecommunication system which enables the alerting of emergency services. In addition, many smart devices also have additional sensors including an accelerometer, video camera and a photo-plethysmography (PPG) sensor which may be useful in the execution of the CPR workflow such as real-time feedback on the presence of vital signs. Such portable or wearable computing devices may therefore be leveraged to support a CPR coordination system, which can be used to assist lay rescuer coordination and intervention whenever or wherever an emergency situation occurs.

Thus, there may be provided a method for assessing and identifying availability and/or suitability of laypersons in the vicinity of an SCA or accident victim who may help the application of CPR. Embodiments may also assess and identify a layperson/bystander and/or their attributes that could adversely affect the delivery of effective CPR. The method also assesses and identifies availability and/or suitability of sensor components comprised in a plurality of portable computing devices identified by a coordinator in an area around said coordinator that comprises the SCA or accident victim. Using such assessment(s), the coordinator can provide better and more accurate cardiopulmonary resuscitation coordination guidance to one or more of the identified devices so that a layperson may be guided to avoid performing (but instead assist another person) so as to ensure safe and effective CPR performance.

Embodiments propose the concept of processing device-attribute information relating to the identified portable computing devices that are identified within an area around a coordinator and that comprises a patient to be treated, and user-attribute information relating to users associated with said portable computing devices. In response to the processed device-attribute information and user-attribute information, sensor functionalities are assigned to sensor components of one or more of the identified portable computing devices, and a cardiopulmonary resuscitation role can be assigned to at least one of the users associated with the identified portable computing devices. Cardiopulmonary resuscitation coordination guidance can then be communicated to at least one of the users in response to the assigned cardiopulmonary resuscitation role(s), and data acquired by the sensor components assigned with a sensor functionality.

The user-attribute information may comprise at least one of: medical qualification information; resuscitation experience information; medical training information; and physical condition information, for instance to assess if a user associated with an identified portable computing devices possesses certain attributes (such as medical training and/or CPR experience) which could help to ensure safe and/or effective CPR performance.

Medical qualification information may, for example, comprise information relating to qualifications and/or educational records of individuals such as qualification(s), level of qualification(s), length of time qualified, experience level, and the like. Resuscitation experience information may, for example, comprise information relating to individuals' past experience of applying CPR such as application frequency, time elapsed since last CPR application or training, success/failure rate, etc. Medical training information may, for example, comprise information relating to medial training or courses completed by individuals such as medical/first aid course(s) completed, level of medical/first aid course(s), time elapsed since course completion, etc. Physical condition information may, for example, comprise information relating to physical attributes of individuals such as age, weight, body mass index, fitness level, physical ability, blood pressure, physical condition(s)/illness(es), vital signs (e.g. heart rate and breathing rate), etc.

Thus, embodiments may enable rapid identification of individuals within a crowd that are trained in CPR. It may also enable the prioritization and coordination of steps taken by more than two individual lay rescuers during CPR.

As such, embodiments may help to prevent lay rescuers from being confused, overwhelmed or disagreeing as to their participation or role(s) when confronted SCA event or emergency situation with multiple bystanders or lay rescuers.

In accordance with the present disclosure, the obtained user-attribute information and device-attribute information is used to assign roles to users holding the portable computing devices in the vicinity of the coordinator and to assign sensor functionalities to the sensor components included in said portable computing devices, thereby building a ubiquitous network to provide CPR coordination guidance to the users so that they can deliver CPR to a patient in a collaborative manner. This task may be subdivided into smaller tasks which collectively performed achieve the complete medical task. In an example, this medical task may be divided into skill-involved tasks and supportive non-skill-involved tasks. For skill-involved tasks, the user-attribute information and device-attribute information is utilized to select users to perform the lead role (i.e., lead CPR rescuer) and the relief/support role(s) (i.e., CPR relief rescuer). User-attribute information such as medical expertise, prior CPR training, recent CPR training etc., may increase a user's likelihood of being assigned a skill-involved role. In addition, device-attribute information will also be used. For example, if the user has a Google Glass able to perform the pulse check and breathing check or if the user possesses a portable computing device with a pulse detection sensor. A user with both CPR experience and a smart device capable of performing specific sensor functionality related to CPR will be more likely to be assigned the lead or support role. For the non-skill-involved task, the user-attribute information and the device-attribute information is used to select users to perform specific roles. Non-skill-involved tasks are assigned to users who do not possess attributes indicating that they have sufficient skill needed to perform CPR, and who possess devices which have appropriate capabilities required to perform the necessary non-skill-involved tasks. For example, a non-skilled user with an outdated smartphone with very limited functionality and who is not young (e.g., over 50 years old), may be assigned to perform crowd control. A non-skilled user with an advanced smartphone may be assigned to document the scene using their device's camera and microphone. A non-skilled user who is young and has a smartphone with GPS will be assigned to retrieve an automated external defibrillator from a nearby location. A non-skilled user who is older in age and possesses a basic smartphone may be assigned to perform communication with the emergency medical service (EMS) dispatcher. In this way all of the skill-involved and non-skill-involved roles required to perform CPR may be more efficiently assigned.

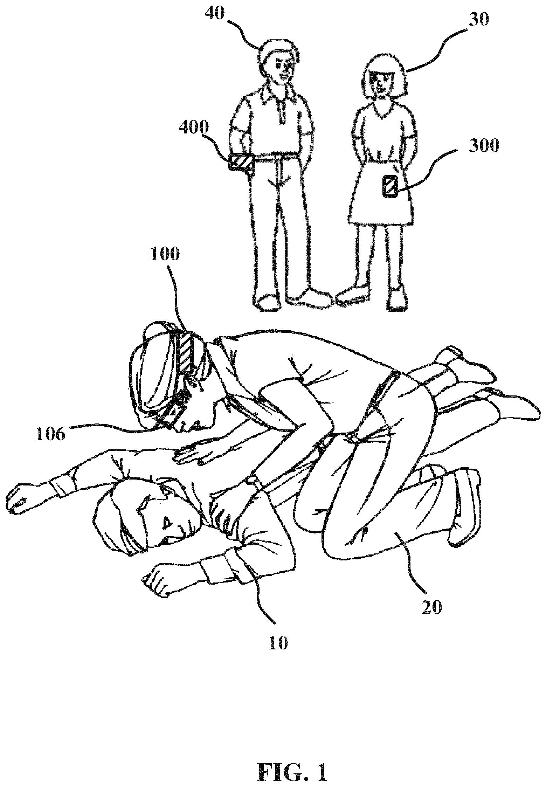

FIG. 1 schematically depicts a first 20, second 30 and third 40 lay rescuers in the vicinity of a victim or patient 10 employing a CPR coordination system according to an embodiment. The CPR coordination system comprises a head-mountable computing device 100 worn by the first lay rescuer 20, and which act as coordinator for the CPR coordination system. The head-mountable computing device 100 provides the first rescuer 20 with CPR guidance in the form of displayed instructions on a display module 106 of the head-mountable computing device 100. By way of non-limiting example, the head-mountable computing device 100 is depicted as smart glasses, but it should be understood that the head-mountable computing device 100 may take any suitable shape as previously explained. The head-mountable computing device 100 typically comprises at least one display module 106, which may be a see-through or transparent display module 106.

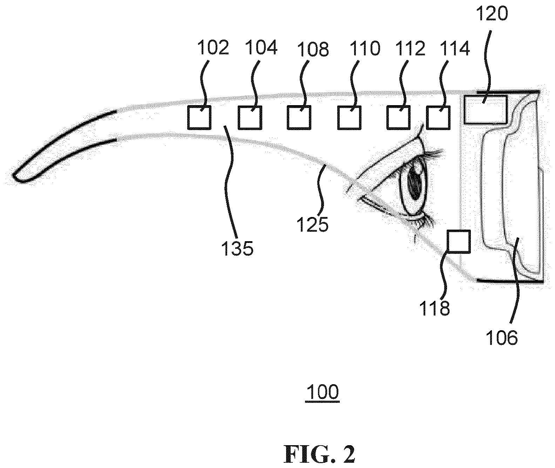

FIG. 2 schematically depicts an example embodiment of such a head-mountable computing device 100 in more detail. In some embodiments, the head-mountable computing device 100 may be adapted to wirelessly communicate with remote components of the CPR coordination system, as will be explained in more detail below. To this end, the head-mountable computing device 100 may include a wireless communication interface 102 for wirelessly communicating with such a remote target. Any suitable wireless communication protocol may be used for any of the wireless communication between the head-mountable computing device 100 and such remote components, e.g., an infrared link, Zigbee, Bluetooth, a wireless local area network protocol such as in accordance with the IEEE 802.11 standards, a 2G, 3G or 4G telecommunication protocol, and so on.

The head-mountable computing device 100 may optionally comprise a further wireless communication interface 104 for wirelessly communicating with a further remote system, e.g. a wireless LAN, through which the head-mountable computing device 100 may access a remote data source such as the Internet, for instance to retrieve data from a remote database as will be explained in more detail below. Alternatively, the head-mountable computing device 100 may include one wireless communication interface that is able to communicate with various remote targets. The data processor 110 may further be adapted to control wireless communication interface 102 and, if present, wireless communication interface 104.

The at least one display module 106 may be under control of a discrete display processor 108, which discrete display processor 108 may be controlled by the data processor 110, for instance receive instructions from the data processor 110 for displaying CPR coordination guidance on the at least one display module 106. Alternatively, the display processor 108 and the data processor 110 may be implemented by a single processor, e.g. a general purpose processor or an application specific integrated circuit (ASIC).

In some embodiments, the head-mountable computing device 100 may be arranged to detect a user instruction and to trigger an operation in response to the detected user instruction, e.g. using at least one further sensor 118 including a motion sensor like a gyroscope or similar in case the user instruction is a head motion, or by including an outward-facing image sensor or camera to capture an image of a gesture-based instruction made by the wearer. Other suitable sensors for such gesture or motion capturing will be apparent to the skilled person.

The data processor 110 may be arranged to recognize a gesture or motion made by its wearer from the captured sensor data and to interpret the recognized gesture or motion as an instruction. Non-limiting examples of such a motion for instance include a turn or nod of the wearer's head. Non-limiting examples of such a gesture for instance include a hand or finger gesture in the field of view through the head-mountable computing device 100, which may be detected in an image captured with an outward facing image sensor 120.

Alternatively or additionally, the at least one further sensor 118 may include a sound sensor, e.g. a microphone, to detect a spoken instruction, wherein the processor 110 may be communicatively coupled to the further sensor in order to process the sensor data and detect the spoken instruction.

The at least one further sensor 118 may additionally or alternatively include an input sensor, e.g. a button or the like for facilitating the wearer of the head-mountable computing device 100 to select a user instruction from a list of options. Such list of options for instance may be displayed on a display module 106 of the head-mountable computing device 100. Such an input sensor may form part of a user interface for receiving input from the user and for communicating outputs to the user. Such a user interface may include, for example, a touchpad, a keypad, buttons, a microphone, a speaker, a display and/or other input or output devices. The data processor 110 may control at least some of the functioning of head-mountable computing device 100 based on input received through the user interface. In some embodiments, any of the at least one further sensors 118 may define or form part of the user interface.

In some embodiments, the head-mountable computing device 100 may further comprise an audio output device 114 such as a loudspeaker or the like for providing the wearer of the head-mountable computing device 100 with audio instructions, e.g. spoken instructions supplementary to the CPR coordination guidance to be displayed on the at least one display module 106. Any suitable audio output device may be used for this purpose. In some embodiments, the display module 106 and/or the audio output device 114 may define or form part of the user interface.

The head-mountable computing device 100 may further comprise a data storage device 112, e.g. for storing the CPR coordination guidance data to be communicated to the user. Any suitable type of data storage may be used, e.g. non-volatile or flash memory, PROM, EEPROM and so on.

The various components of the head-mountable computing device 100 may be integrated in the device in any suitable manner, such as integrated in a part 135 of a mounting frame of the head-mountable computing device 100 by way of non-limiting example.

FIG. 2 schematically depicts an example embodiment of the head-mountable computing device 100 in which the device comprises a single display module 106 only, which single display module 106 may be arranged to be observable by a single eye of the wearer of the head-mountable computing device 100. It should be understood that this is by way of non-limiting example only, and that it is equally feasible to provide a head-mountable computing device 100 in which prospective display modules 106 are provided for each eye of the rescuer 20. The at least one display module 106 is typically arranged such that a wearer of the head-mountable computing device 100, i.e. the rescuer, can observe an image displayed on the at least one the display module 106. Preferably, the at least one display module 106 is a see-through or transparent display module such that the wearer can observe at least a part of a field of view through the display module 106, such that the wearer can wear the head-mountable computing device 100 whilst performing CPR on the victim 10.

The at least one display module 106 may be provided in any suitable form, such as a transparent lens portion. Alternatively, the head-mountable computing device 100 may comprise a pair of such lens portions, i.e. one for each eye as explained above. The one or more transparent lens portions may be dimensioned such that substantially the entire field of view of the wearer is obtained through the one or more transparent lens portions. For instance, the at least one display module 106 may be shaped as a lens to be mounted in a frame 125 of the head-mountable computing device 100. It will be understood that the frame 125 may have any suitable shape and may be made of any suitable material, e.g. a metal, metal alloy, plastics material or combination thereof. Several components of the head-mountable computing device 100 may be mounted in the frame 125, such as in a component housing 135 forming part of the frame 125. The component housing 135 may have any suitable shape, preferably an ergonomic shape that allows the head-mountable computing device 100 to be worn by its wearer in a comfortable manner.

As also shown in FIG. 1, the CPR coordination system further comprises second 300 and third 400 portable computing devices associated with the second 30 and third 40 lay rescuers, respectively. More specifically, the second portable computing device 300 comprises a smartphone (commonly referred to as a mobile phone), and the third mobile computing device 400 comprises a smart watch.

The second portable computing device 300 may be adapted to wirelessly communicate with remote components of the CPR coordination system, similarly to the head-mountable computing device 100. To this end, the second portable computing device 300 may include a wireless communication interface for wirelessly communicating with such a remote target. Again, any suitable wireless communication protocol may be used for any of the wireless communication. The head-mountable computing device 100, as coordinator in the CPR coordination system, may optionally comprise a further wireless communication interface for wirelessly communicating with a further remote system, e.g. a wireless LAN, through which the second portable computing device 300 may access a remote data source such as the Internet, for instance to send and/or retrieve data to/from a remote database. Alternatively, the second portable computing device 300 may include one wireless communication interface that is able to communicate with various remote targets. In some embodiments, the second portable computing device 300 may be arranged to detect a user instruction and to trigger an operation in response to the detected user instruction. Alternatively or additionally, the second portable computing device 300 may include a sound sensor, e.g. a microphone, to detect a spoken instruction, wherein the second portable computing device 300 may be adapted to process the sensor data and detect the spoken instruction. The second portable computing device 300 may additionally or alternatively include an input sensor, e.g. a button or the like for facilitating the user of the second portable computing device 300 to select a user instruction from a list of options. Such an input sensor may form part of a user interface for receiving input from the user and for communicating outputs to the user. Such a user interface may include, for example, a touchpad, a keypad, buttons, a microphone, a speaker, a display and/or other input or output devices. In some embodiments, a second portable computing device 300 may further comprise an audio output device such as a loudspeaker or the like for providing the user of the second portable computing device 300 with audio instructions, e.g. spoken instructions supplementary to the CPR coordination guidance to be displayed on the at least one display module 106. Any suitable audio output device may be used for this purpose.

The second portable computing device 300 may further comprise a data storage device, e.g. for storing CPR coordination guidance data to be communicated to the user. Any suitable type of data storage may be used, e.g. non-volatile or flash memory, PROM, EEPROM and so on.

Similarly, the third portable computing device 400 may be adapted to wirelessly communicate with remote components of the CPR coordination system, similarly to the head-mountable computing device 100. To this end, the third portable computing device 400 may include a wireless communication interface for wirelessly communicating with such a remote target. Again, any suitable wireless communication protocol may be used for any of the wireless communication. The head-mountable computing device 100, as coordinator in the CPR coordination system, may optionally comprise a further wireless communication interface for wirelessly communicating with a further remote system, e.g. a wireless LAN, through which the third portable computing device 400 may access a remote data source such as the Internet, for instance to send and/or retrieve data to/from a remote database. Alternatively, the third portable computing device 400 may include one wireless communication interface that is able to communicate with various remote targets. In some embodiments, the third portable computing device 400 may be arranged to detect a user instruction and to trigger an operation in response to the detected user instruction. Alternatively or additionally, the third portable computing device 400 may include a sound sensor, e.g. a microphone, to detect a spoken instruction, wherein the third portable computing device 400 may be adapted to process the sensor data and detect the spoken instruction. The third portable computing device 400 may additionally or alternatively include an input sensor, e.g. a button or the like for facilitating the user of the third portable computing device 400 to select a user instruction from a list of options. Such an input sensor may form part of a user interface for receiving input from the user and for communicating outputs to the user. Such a user interface may include, for example, a touchpad, a keypad, buttons, a microphone, a speaker, a display and/or other input or output devices. In some embodiments, third portable computing device 400 may further comprise an audio output device such as a loudspeaker or the like for providing the user of the third portable computing device 400 with audio instructions, e.g. spoken instructions supplementary to the CPR coordination guidance to be displayed on the at least one display module 106. Any suitable audio output device may be used for this purpose.

The third portable computing device 400 may further comprise a data storage device, e.g. for storing CPR coordination guidance data to be communicated to the user. Any suitable type of data storage may be used, e.g. non-volatile or flash memory, PROM, EEPROM and so on.

In an embodiment, a sensor component included in the third portable computing device 400 may comprise an integrated sensor for detecting vital signs of its user 40. In another example, the sensor component of the third portable computing device 400 may comprise a forward facing camera 120 for capturing an image of (the face of) the victim 10, which image (or sequence of images) may be processed by the data processor 110 in order to extract vital signs information (e.g. breathing characteristics) from the image (or sequence of images), e.g. using an application from the memory for extracting vital signs information from a captured image, as is well-known per se.

Alternatively, one or more dedicated vital sign sensors may be incorporated in the sensor component of the third portable computing device 400. For example, such an external sensor may be integrated within the smart device to allow for accurate monitoring of the vital signs of the user 40. In the context of the present application, vital signs include at least one of: a breathing pattern or rhythm; blood pressure, body temperature; hear/pulse rate and a pulse rhythm. It should be understood for the avoidance of doubt that a pulse rhythm is not necessarily detected in the pulse of a user 40, but may be detected at any suitable part of the body of the user as is well-known per se. As will be explained in more detail below, data captured by the one or more sensors may be processed and used to generate CPR coordination guidance.

In an embodiment, the sensor component of at least one of the portable computing devices 100,300, 400 comprises a sensor for sensing a location of the device. Such a sensor may for instance comprise a global positioning system (GPS) unit for determining the location of the device, i.e. the location of the sensor. In an embodiment, the sensor may be integrated in the head-mountable computing device 100, for example. However, in the example of FIG. 1, the sensor is external to the head-mountable computing device 100 and is instead integrated into the third portable computing device 400 worn on the wrist of the third lay rescuer 40, such as a smart watch or the like.

As will be explained in more detail below, data captured by such a sensor can be processed and used to take account of lay rescuer's location during an SCA event or an emergency situation, thereby enabling the coordinator of the CPR coordination guidance to take appropriate action in view of a lay rescuer's location, for example.

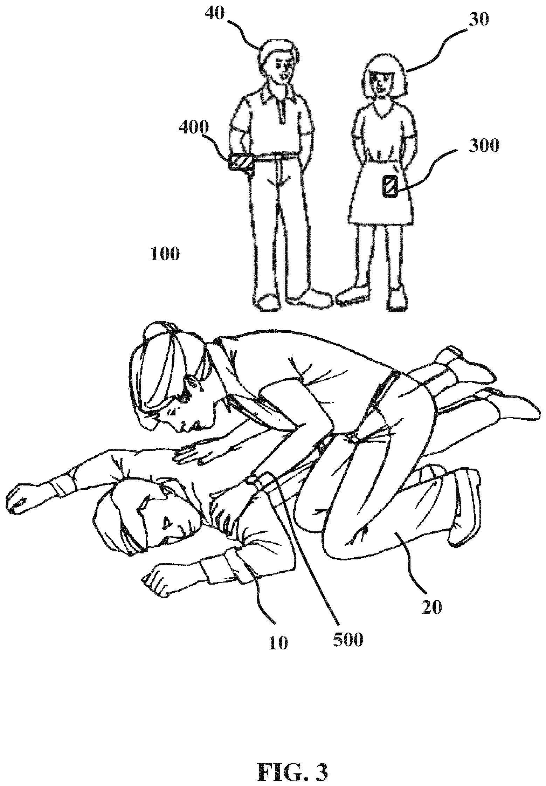

It is not necessary for the first computing device 100 to be head-mountable computing device 100. FIG.3 schematically depicts an alternative embodiment of a portable computing device 500 associated (e.g. used by) the first lay rescuer 20), wherein the portable computing device 500 is a smartwatch 500.

At this point it is noted that the CPR guidance system is able to be created in situ, using ubiquitous smart devices that are in the possession, e.g. worn or carried by a plurality of lay rescuers 20, 30, 40. Given the rapid proliferation of such smart devices in everyday life, this therefore means that there is an excellent chance that such a CPR coordination system according to an embodiment may be created in situ by identifying suitable sensor components thereof at the scene of a SCA event and incorporating the identified components in the CPR coordination system, e.g. by establishing wireless links between the identified portable computing and/or a remote processing resource.

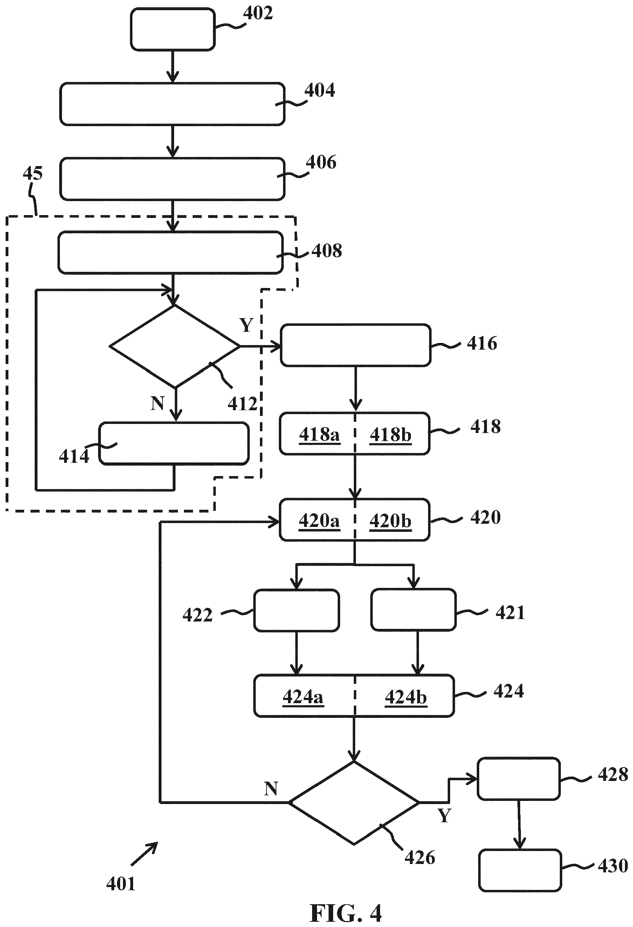

This will be explained in more detail with the aid of FIG. 4, which depicts a flowchart of an example embodiment of a method 401 for providing CPR coordination guidance using an embodiment of the proposed CPR coordination system. In a preferred embodiment, the method 401 comprises a first branch 45 in which portable computing devices 300, 400 in the vicinity of a coordinator (e.g. portable computing device 100) associated with the rescuer 20 establish one or more connections with each other, and a second branch in which the CPR coordination system is used to assign sensor functionalities to the to the sensor component of at least one of the identified portable computing devices and a cardiopulmonary resuscitation role to at least one user associated with said identified portable computing devices in response to processed device-attribute information and user-attribute information. It is noted for the avoidance of doubt that the first branch 45 may be omitted from the method 401 in case of the portable computing devices 300, 400 in the vicinity of the coordinator 100 (and the rescuer 20 associated with it) communicating to a remote server or processing resource, for example.

The method 40 may start in step 402 by the discovery of a suspected victim of a SCA event by the first lay rescuer 20 wearing the head-mountable computing device 100. The first lay rescuer 20 may activate a CPR assistance mode of the head-mountable computing device 100, e.g. by providing the device 100 with a command as explained above that can be recognized by the device 100 as an instruction to activate the CPR administration mode, which may prompt the head-mountable computing device 100 to generate a first responder call to a remote server associated with emergency services in step 404, in order to direct the emergency services to the location of the victim 10. To this end, the first responder call may be an automated call comprising global positioning information, which information may be obtained from a global positioning unit within the head-mountable device 100 or a global positioning unit in communication with the head-mountable device 100, e.g. a smart device such as a smart phone or a smart watch comprising a global positioning unit, which smart device is wirelessly linked to the head-mountable device 100. Alternatively, the first responder call is generated by the first lay rescuer 20, e.g. by issuing an instruction to the head-mountable computing device 100 to generate the call.

Next, the method 40 progresses to step 406 in which the head-mountable computing device 100, as coordinator of the CPR coordination system, searches in its vicinity (i.e. within a predetermined surrounding area) which includes the victim 10 for portable computing devices having a communication interface (for communicating information to the head-mountable computing device 100 and/or a remote processing resource for example) and a sensor component (for acquiring data of the user of said portable computing device, the patient and/or the area around the coordinator). Such devices may for instance be detected by identifying a wireless signal generated by these devices or by identifying a global positioning tracking signal, e.g. a GPS tracker signal, a GRS signal, a GLONASS signal or the like. The available devices may be recognized in any suitable manner, for instance by recognizing the device type and make (product number), e.g. from an interrogation of the devices in the vicinity of the coordinator associated with the first lay rescuer 20, and comparing the identified device with a stored database of known devices with the desired functionality, or by requesting the smart devices in the vicinity of the coordinator 100 to indicate availability.

Next, in step 408 the head-mountable computing device 100 attempts to establish a wireless connection using any suitable wireless communication protocol as previously explained to the identified devices 300, 400 to be incorporated in the CPR coordination system. In step 412 it is checked if the wireless connections between the identified devices 300,400 and the head-mountable computing device 100 have been established. If for some reason some of the wireless connections have not been established, the method 401 proceeds to step 414 in which the first lay rescuer 20 is alerted that some of the wireless connections could not be established, such that the rescuer 20 can attempt to adjust the affected devices, for instance by instructing the enablement of a wireless communication mode of these devices 300,400. This may involve asking a bystander 30, 40 to activate the wireless communication mode in case the device has been provided by the bystander 30,40. The alert may be displayed on the at least one display module 106 of the head-mountable computing device 100. The method 401 then returns to step 412 to check if all devices have established a wireless communication link with the head-mountable computing device 100.

Once all devices are wirelessly connected to the head-mountable computing device 100, the method 401 proceeds to step 416 in which device-attribute information relating to the portable computing devices 300, 400 and user-attribute information relating to the users 30,40 associated with said devices 300,400 is obtained. By way of example, the user-attribute information may comprise at least one of: medical qualification information; resuscitation experience information; medical training information; and physical condition information, for instance for providing information useful for determining is certain user 30,40 is suitable or unsuitable for performing CPR. Also, the user-attribute information may enable the assessment and identification of individuals which could adversely affect the delivery of effective CPR.

The user-attribute information relating to a user may, for instance, be stored on the portable computing device used by that user. Thus, the second portable computing device 300 may store data user-attribute information relating to the second lay person 30, and the third portable computing device 400 may store data user-attribute information relating to the third lay person 40. Furthermore, additional user-attribute information about a user may be captured from one or more sensors for monitoring vital signs which are integrated in the sensor component of the user's portable computing device. This may, for instance, be accomplished by implementing a heart rate and/or breathing classification algorithm which analyses vital signs of the user.

Similarly, by way of example, the device-attribute information may comprise at least one of: sensor components available on the portable computing device, battery specifications, model number information (e.g. device age), supported wireless communication standards, and other information for determining if certain portable computing devices 300,400 are suitable or unsuitable for performing certain sensor functionalities.

Such device-attribute information and user-attribute information may be communicated to the head-mountable computing device 100 via the established wireless communication links.

Next, in step 418, the obtained device-attribute information and user-attribute information is processed, and such processing may be assisted by processing undertaken via a distributed processing environment (such as the 'cloud' for example). Also, additional device-attribute information and/or user-attribute information may be provided from other services, e.g. medical history information stored in a database or portable computing device stored in a database such as the product certification database of the Federal Communications Commission, and taken into account by the processing. Step 418 is subdivided into two sub-steps, which may occur at the same time or one be started or finished before the other. In sub-step 418a, various types of user-attribute information, including: medical qualification information; resuscitation experience information; medical training information; CPR training information, and physical condition information may be processed by the data processor 110 to identify lay rescuers who may positively or negatively influence the delivery of effective CPR, e.g., a highly skilled medical professional, an untrained individual, a first-aid-trained individual, a person with CPR experience or training, etc. In sub-step 418b various types of device-attribute information may be processed by the data processor 110 to identify portable computing devices including sensor components which may be suitable for implementing a plurality of sensor functionalities required for providing CPR guidance.

In response to the processed user-attribute information, the method proceeds to step 420, which is again subdivided in two sub-steps that can be carried out sequentially in any order or simultaneously in parallel. In sub-step 420a the data processor 110 determines one or more indicators of the suitability of each user 20,30,40 for performing cardiopulmonary resuscitation on the victim 10. By way of example, if user-attribute information is indicative that a user has little to no medical/first-aid knowledge or experience, an indicator may be extracted to indicate that the user is of low (or zero) suitability for performing cardiopulmonary resuscitation. The indicator may even be indicative that such a user (e.g. a bystander) should not be permitted to intervene. Also, obtained user-attribute information may be indicative of a user's willingness or readiness to participate in CPR performance. Vital signs of the user and/or the user's indicated willingness or readiness can be accounted for when determining an indicator. Moreover, to increase the reliability of an indicator, both vital signs and user response(s) can be used together to confirm/qualify a user's indicated willingness or readiness to perform CPR. Thus, generated or extracted indicators may be based on a range of user-attribute information provided by the identified portable computing devices 300,400 and/or other user-attribute information provided by one or more databases. Such determination of indicators may be done either in part or entirely on the `Cloud' (e.g. via a distributed processing environment).

In sub-step 420b the data processor 110 determines, for each respective sensor functionality of said plurality of sensor functionalities, a respective indicator of the sensor component of the identified portable computing devices 300,400 indicative of the suitability of the sensor components for performing said respective sensor functionality required for the administration of CPR to the victim 10.

The method then proceeds to steps 421 and 422, which although depicted in Fig. 3 as taking place in parallel, they could alternatively be implemented sequentially in any order.

In step 421, for each sensor functionality of the plurality of sensor functionalities, the coordinator 100 assigns a respective sensor functionality of said plurality to the sensor component of at least one of the identified portable computing devices 300,400 in response to the processed device-attribute information and user-attribute information. The assigned sensor functionalities, is based on the determined indicators. For example, for each respective sensor functionality, the respective determined indicator may be used to rank or prioritize the sensor components of the identified devices according to their suitability.

In step 422, the coordinator 100 assigns a cardiopulmonary resuscitation role is assigned to at least one of the users 30,40 associated with the identified portable computing devices 300, 400 in response to the processed device-attribute information and user-attribute information. The assigned role(s), is based on the determined indicators. For example, the determined indicators for the users may be used to rank or prioritize the users in order of their suitability for CPR performance. This may be done using a suitability index-based decision model for example. One or more cardiopulmonary resuscitation roles may then be assigned to the users based on such ranking or prioritization, such as primary user role for performing cardiopulmonary resuscitation and one or more assistant user roles for assisting the primary user. Lowest ranked/prioritized may be assigned non-CPR roles, or non-participating roles, but instead guided to a safe nearby location to await emergency services arrival (e.g. using location information obtained by a GPS tracker of the system).

The method proceeds to step 424, which is again subdivided into two sub-steps. In sub-step 424a the assigned role(s) is/are communicated to at least one of the lay rescuers 30,40 via at least one of the portable computing devices 300,400. For example, where the primary user role is assigned to the first lay rescuer 20 associated with the coordinator, the first lay rescuer 20 is instructed to perform CPR on the victim 10, whereas the second lay rescuer 30 (that is assigned an assistant role) is instructed to control the third lay rescuer 40 (e.g. by directing then to a certain location away from the scene of the SCA or accident) and await the arrival of emergency services. Similarly, in sub-step 424b the assigned sensor functionalities are communicated by the coordinator 100 to at least one of the portable computing devices 300,400.

The method then proceeds to step 426 in which it is checked if the second 30 and third 40 lay rescuers accept the communicated coordination guidance, or if the second 300 and third 400 portable computing devices accept the communicated sensor functionality for their sensor components. This for instance may be checked by prompting the second 30 and third 40 lay rescuers to indicate their acceptance of the communicated coordination guidance. For example, second lay-rescuer's reluctance to administer CPR (e.g. due to being physically tired, injured or disabled) may be indicated by the second lay-rescuer user by indicating non-acceptance of the assigned primary user role. Such indication may be made by the second lay-rescuer providing the device 300 with a response command as explained above that can be recognized by the device 300 as response information for indicating acceptance of an assigned cardiopulmonary resuscitation role and communicated to the coordinator 100. Similarly, a portable computing device 300,400 may communicate to the coordinator 100 sensor functionality response information for indicating acceptance of a sensor functionality assigned to the sensor component of said device.

If it is determined that the one or more of the lay rescuers do not accept the communication coordination guidance, or that one or more of the portable computing devices do not accept the assigned sensor functionality, the method 401 returns to step 420 wherein the data processor 110 once again determines one or more updated or adjusted indicators of the suitability of each user 30,40 for performing cardiopulmonary resuscitation on the victim 10 and/or the suitability of the sensor component of the portable computing devices 300,400 to perform a sensor functionality. The updated indicators of the suitability may be adjusted, for example, based on the response information that was provided as a result of a user indicating non-acceptance or acceptance of an assigned cardiopulmonary resuscitation role.

If, in step 426, it is determined that the lay rescuers accept the communicated coordination guidance and that the portable computing devices accept the communicated sensor functionality for their respective sensor components, then the method proceeds to step 428 in which data acquired by at least one of the sensor components assigned with a sensor functionality is transmitted to the coordinator 100. The acquired data may relate, for example, to a vital sign of the user 30, 40 of the portable computing device, or to a vital sign of the victim 10 and/or to a hazard in the area around the coordinator 100;

Finally, the method proceeds to step 430 in which the data processor 110 triggers the communication of specific coordination guidance from the coordinator 100 to the portable computing devices 300, 400 based on the assigned cardiopulmonary resuscitation role(s) and the acquired data. For example, the guidance information is presented with CPR guidance on the at least one display module 406, which typically includes guidance regarding the tasks to be performed for an assigned role. For a primary user role, the guidance may include instructions detailing the way in which chest compressions and rescue breaths should be administered, e.g. information about the chest compression depth and frequency, information about when to administer how many rescue breaths and so on. For an assistant role, the guidance may include instructions detailing where to direct other persons to for their safety, or information about when the emergency services may be expected to arrive.

In some embodiments, the method 401 may then loop back to step 406 to perform repeated identification of portable computing devices within the vicinity (e.g. within a predetermined range) of the coordinator 100. In this manner, the method 401 may continue to seek for new users who may be better suited or positioned to one or more CPR roles and/or for devices with sensor modules better suited to perform a sensor functionality required for CPR. This may continue until the victim 10 exhibits a return to spontaneous circulation, e.g. exhibits spontaneous breathing and a pulse as detected in step 428, or until emergency services arrive on the scene to take over, upon which the method 401 may terminate by the rescuer 20 terminating CPR and placing the victim 10 in the recovery position.

At this point, it is noted that the above described embodiment of the method 401 is merely an example embodiment of this method and that several extensions thereto and/or variations thereon will be immediately apparent to the skilled person.

For example, the method 401 may be extended by the detection of a portable defibrillation device in the vicinity of the coordinator 100 and the victim 10, e.g. in the procession of one of the bystanders, which detection for instance may take place in step 406 of the method 401 by way of non-limiting example, in which case the CPR coordination method may be extended by communicating instructions for lay rescuer on how to use the defibrillation device in the CPR workflow. For instance, user instructions for using the defibrillation device may be directly transferred from the defibrillation device to the head-mountable computing device 100 for displaying on the at least one display module 106.

Other suitable extensions and variations to the above disclosed embodiments will be apparent to the skilled person.

For example, embodiments may be adapted to identify all individuals in a crowd of bystanders, wearing smart watches, who have registered their previous CPR/medical training and or prior resuscitation experience in a cloud-based database. Different roles may be assigned to bystanders in a crowd based on their level of training and experience. For instance, a lead lay responder may be assigned based on the level of CPR/medical training and prior resuscitation experience. A second individual, without training, may be assigned to contact and maintain communication with emergency services throughout the CPR process. A third individual, with CPR training, may be assigned to assist the lead rescuer in providing CPR, and to provide relief when the lead lay responder becomes fatigued. A fourth untrained individual may be assigned to locate and retrieve the nearest AED. A fifth untrained individual may be assigned to record video footage of the resuscitation process which can be transmitted later to BLS and ALS providers for documentation of the entire resuscitation process. Additional individuals in the crowd with smart watches and without CPR training may be assigned to perform crowd control duties and to ensure that a clear access route is maintained for BLS and ALS responders.

Embodiments may therefore help ensure that individuals in a crowd will not be hesitant to intervene to help a victim and will not be confused over the role that they can play in helping the victim. In so doing, the CPR workflow may be optimized as there will be no delay in intervention and no duplication of effort.

The proposed concept has the advantage that a network of portable computing device with monitoring and/or communication functions can be easily transformed into a CPR workflow coordination system, which could be used to optimize CPR delivery by a crowd of bystanders whenever or wherever an emergency situation occurs.

Aspects of the present invention are embodied as a cardiopulmonary resuscitation guidance method or system at least partially embodied by a portable computing device or distributed over separate entities including a portable computing device. Aspects of the present invention take the form of a computer program product embodied in one or more computer-readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present disclosure by execution on the processor 110 may be written in any combination of one or more programming languages, including an object oriented programming language such as Java, Smalltalk, C++ or the like and conventional procedural programming languages, such as the "C" programming language or similar programming languages. The program code may execute entirely on the processor 110 as a stand-alone software package, e.g. an app, or may be executed partly on the processor 110 and partly on a remote server. In the latter scenario, the remote server may be connected to the head-mountable computing device 100 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the disclosure. It will be understood that each block of the flowchart illustrations and/or block diagrams, and combinations of blocks in the flowchart illustrations and/or block diagrams, can be implemented by computer program instructions to be executed in whole or in part on the data processor 110 of the cardiopulmonary resuscitation coordination system including portable computing device, such that the instructions create means for implementing the functions/acts specified in the flowchart and/or block diagram block or blocks. These computer program instructions may also be stored in a computer-readable medium that can direct the cardiopulmonary resuscitation guidance system including the portable computing device to function in a particular manner.

The computer program instructions may be loaded onto the display processor 108 and/or the data processor 110 to cause a series of operational steps to be performed on the display processor 108 and/or the data processor 110, to produce a computer-implemented process such that the instructions which execute on the display processor 108 and/or the data processor 110 provide processes for implementing the functions/acts specified in the flowchart and/or block diagram block or blocks. The computer program product may form part of a cardiopulmonary resuscitation coordination system including a portable computing device, e.g. may be installed on the cardiopulmonary resuscitation guidance system.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The disclosure can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A cardiopulmonary resuscitation coordination method (401) between: a portable coordinator device (100) associated with a first user; and a plurality of portable computing devices (300, 400), the portable coordinator device comprising a communication interface arranged to communicate information via a communication link, the method comprising:
identifying (406) a plurality of portable computing devices (300, 400) located within an area around the portable coordinator device (100), said area comprising a patient (10) to be treated, each of the plurality of portable computing devices being associated with a respective user (30, 40) and comprising a communication interface, arranged to communicate information via the communication link, and a sensor component arranged to acquire data of the user (30, 40) of said portable computing device, the patient (10) and/or the area around the portable coordinator device (100);
processing (418a) user-attribute information relating to the users associated with the identified portable computing devices so as to determine a respective suitability indicator for each of the users associated with the identified portable computing devices, said respective suitability indicator being indicative of the suitability of the user for performing cardiopulmonary resuscitation on the patient;
processing (418b) device-attribute information relating to the identified portable computing devices, wherein processing (418b) device-attribute information comprises, for each respective sensor functionality of a plurality of sensor functionalities, extracting a respective functionality indicator (420b) of the sensor component of the identified portable computing devices from said device-attribute information, said respective functionality indicator being indicative of the suitability of the sensor component of the identified portable computing device for performing said respective sensor functionality;
for each sensor functionality of the plurality of sensor functionalities, the portable coordinator device assigning (421) a respective sensor functionality to the sensor component of at least one of the identified portable computing devices based on the suitability indicators and functionality indicators;
the portable coordinator device assigning (422) a cardiopulmonary resuscitation role to at least one of the users associated with the identified portable computing devices based on the above suitability indicators and functionality indicators;
communicating (428) data acquired by at least one of the sensor components assigned with a sensor functionality from the respective identified device or devices to the portable coordinator device via the communication link, the data relating to at least one of: a vital sign of a user associated with the respective identified device; a vital sign of the patient; and a hazard in the area around the portable coordinator device (100); and
communicating (430) specific cardiopulmonary resuscitation coordination guidance from the portable coordinator device to at least one of the identified devices via the communication link in response to the at least one assigned cardiopulmonary resuscitation role and the acquired data.

2. The method of claim 1, wherein the step of processing (418b) device-attribute information further comprises: prioritizing, for each respective sensor functionality, the sensor components of the identified portable computing devices based on the respective extracted functionality indicator.

3. The method of any of claims 1 to 2, wherein said device-attribute information comprises at least one of:
sensor components available on the portable computing device;
battery specifications;
model number information; and
supported wireless communication standards.

4. The method of any of claims 1 to 3, wherein the step of processing user-attribute information comprises:
accessing (416) a database of user-attribute information and extracting suitability indicators (420a) for each of the users associated with the identified portable computing devices from said user-attribute information.

5. The method of claim 4, wherein the step of processing (418a) user-attribute information further comprises: prioritizing the users based on the extracted suitability indicators.

6. The method of any of claims 1 to 5, wherein said user-attribute information comprises at least one of:
medical qualification information;
resuscitation experience information;
medical or cardiopulmonary resuscitation training information; and
physical condition or ability information.

7. The method of any of the preceding claims, further comprising the step of:
capturing (416) user-attribute information from the sensor component of an identified portable computing device,
wherein said sensor component optionally comprises at least one of: a sensor for monitoring vital signs of the user associated with the identified portable computing device; and a sensor adapted to capture an indicator in the form of a response of the user associated with the identified portable computing device to one or more questions presented to the user.

8. The method of any of the preceding claims, wherein the step of communicating (430) cardiopulmonary resuscitation coordination guidance comprises:
communicating first instructions for performing cardiopulmonary resuscitation via the communication link to a first portable computing device, the user associated to the first portable computing device being a primary user; and
communicating second instructions for performing cardiopulmonary resuscitation via the communication link to one or more portable computing devices other than the first portable computing device, the respective user or users associated to said one or more portable computing devices being an assistant user or users for assisting to the primary user.

9. The method of any of the preceding claims, further comprising:
the portable coordinator device (100) receiving sensor functionality response information from an identified portable computing device (300, 400) for indicating acceptance of a sensor functionality assigned to the sensor component of said identified device;
processing said sensor functionality response information; and
communicating adjusted cardiopulmonary resuscitation coordination guidance from the portable coordinator device (100) to at least one of the identified devices (300, 400) via the communication link in response to processed sensor functionality response information and the acquired data.

10. The method of any of the preceding claims, further comprising:
the portable coordinator device (100) receiving role response information from an identified portable computing device (300, 400) for indicating acceptance of an assigned cardiopulmonary resuscitation role in response to the cardiopulmonary resuscitation coordination guidance communicated to said identified device;
processing said role response information; and
communicating adjusted cardiopulmonary resuscitation coordination guidance from the portable coordinator device (100) to at least one of the identified devices (300, 400) via the communication link in response to processed role response information and the acquired data.

11. The method of any of the preceding claims, wherein each of the plurality of portable computing devices (100, 300, 400) comprises at least one of:
a smartphone;
a smartwatch;
a tablet computer;
automatic external defibrillator; and
a head-mountable computing device having a display module arranged to be viewed by a wearer of the head-mountable computing device when wearing the device.

12. The method of any of the preceding claims, wherein at least one of the identified portable computing devices (300, 400) comprises a location sensor for detecting the location of the portable computing device,
and wherein the method further comprises:
sending (404) a distress signal to a remote server, said distress signal including location information indicative the detected location of the portable computing device.

13. A computer program product comprising a computer-readable medium embodying computer program code for implementing the method of any of claims 1 to 12 when executed on a processor (110) of a portable coordinator device (100) associated with a first user.

14. A cardiopulmonary resuscitation coordination system including the computer program product of claim 13, a portable coordinator device (100) associated with a first user, and a plurality of portable computing devices (300, 400) located within an area around the portable coordinator device, said area comprising a patient (10) to be treated, each of the plurality of portable computing devices being associated with a respective user (30, 40) and comprising a communication interface arranged to communicate information via a communication link, and a sensor component arranged to acquire data of the user (30, 40) of said portable computing device, the patient (10) and/or the area around the portable coordinator device (100).

## Patentansprüche

1. Koordinierungsverfahren für kardiopulmonale Wiederbelebung (401) zwischen: einer tragbaren Koordinationsvorrichtung (100), die mit einem ersten Benutzer assoziiert ist; und einer Vielzahl von tragbaren Rechenvorrichtungen (300, 400), wobei die tragbare Koordinationsvorrichtung eine Kommunikationsschnittstelle umfasst, die angeordnet ist, um Informationen über eine Kommunikationsverbindung zu übermitteln, das Verfahren umfassend: Identifizieren (406) einer Vielzahl von tragbaren Rechenvorrichtungen (300, 400), die sich in einem Bereich um die tragbare Koordinationsvorrichtung (100) befinden, der Bereich umfassend einen zu behandelnden Patienten (10), wobei jede der Vielzahl von tragbaren Rechenvorrichtungen mit einem jeweiligen Benutzer (30, 40) assoziiert ist und eine Kommunikationsschnittstelle, die angeordnet ist, Informationen über die Kommunikationsverbindung zu übermitteln, und eine Sensorkomponente, die angeordnet ist, um Daten des Benutzers (30, 40) der tragbaren Rechenvorrichtung, des Patienten (10) und/oder des Bereichs um die tragbare Koordinationsvorrichtung (100) zu erfassen; Verarbeiten (418a) von Benutzerattribut-Informationen, die sich auf die Benutzer beziehen, die mit den identifizierten tragbaren Rechenvorrichtungen assoziiert sind, um einen jeweiligen Eignungsindikator für jeden der Benutzer zu bestimmen, die mit den identifizierten tragbaren Rechenvorrichtungen assoziiert sind, wobei der jeweilige Eignungsindikator indikativ für eine Eignung des Benutzers zum Ausführen einer kardiopulmonalen Wiederbelebung an dem Patienten ist; Verarbeiten (418b) von Vorrichtungsattribut-Informationen, die sich auf die identifizierten tragbaren Rechenvorrichtungen beziehen, wobei ein Verarbeiten (418b) von Vorrichtungsattribut-Informationen für jede jeweilige Sensorfunktion einer Vielzahl von Sensorfunktionen ein Extrahieren eines jeweiligen Funktionsindikators (420b) der Sensorkomponente der identifizierten tragbaren Rechenvorrichtungen aus den Vorrichtungsattribut-Informationen umfasst, wobei der jeweilige Funktionsindikator indikativ für eine Eignung der Sensorkomponente der identifizierten tragbaren Rechenvorrichtung zum Ausführen der jeweiligen Sensorfunktion ist; wobei für jede Sensorfunktion der Vielzahl von Sensorfunktionen, die tragbare Koordinationsvorrichtung der Sensorkomponente von mindestens einer der identifizierten tragbaren Rechenvorrichtungen basierend auf der Eignungsindikatoren und der Funktionsindikatoren eine entsprechende Sensorfunktion zuweist (421); wobei die tragbare Koordinationsvorrichtung mindestens einem der Benutzer, die mit den identifizierten tragbaren Rechenvorrichtungen assoziiert sind, basierend auf den obigen Eignungsindikatoren und Funktionsindikatoren eine Rolle bei der kardiopulmonalen Wiederbelebung zuweist (422); Übermitteln (428) von Daten, die von mindestens einer der Sensorkomponenten, die mit einer Sensorfunktion assoziiert ist, von der jeweiligen identifizierten Vorrichtung oder den Vorrichtungen erfasst werden, an die tragbare Koordinationsvorrichtung über die Kommunikationsverbindung, wobei sich die Daten auf mindestens eines von Folgenden beziehen: ein Vitalzeichen eines Benutzers, der mit der jeweiligen identifizierten Vorrichtung assoziiert ist; ein Vitalzeichen des Patienten; und eine Gefahr im Bereich um die tragbare Koordinationsvorrichtung (100); und Übermitteln (430) spezifischer Anleitungen zur Koordination der kardiopulmonalen Wiederbelebung von der tragbaren Koordinationsvorrichtung an mindestens eine der identifizierten Vorrichtungen über die Kommunikationsverbindung als Reaktion auf die mindestens eine zugewiesene Rolle bei der kardiopulmonalen Wiederbelebung und die erfassten Daten.

2. Verfahren nach Anspruch 1, wobei der Schritt eines Verarbeitens (418b) von Vorrichtungsattribut-Informationen fernes Folgendes umfasst: Priorisieren der Sensorkomponenten der identifizierten tragbaren Rechenvorrichtungen für jede jeweilige Sensorfunktion basierend auf dem jeweiligen extrahierten Funktionsindikator.

3. Verfahren nach einem der Ansprüche 1 bis 2, wobei die Vorrichtungsattribut-Informationen mindestens eines von Folgenden umfassen:
Sensorkomponenten, die auf de tragbaren Rechenvorrichtung verfügbar sind;
technische Batteriedaten;
Modellnummer-Informationen; und
unterstützte drahtlose Kommunikationsstandards.

4. Verfahren nach einem der Ansprüche 1 bis 3, wobei der Schritt eines Verarbeitens von Benutzerattribut-Informationen Folgendes umfasst:
Zugreifen (416) auf eine Datenbank mit Benutzerattribut-Informationen und Extrahieren von Eignungsindikatoren (420a) für jeden der mit den identifizierten tragbaren Rechenvorrichtungen assoziierten Benutzer aus den Benutzerattribut-Informationen.

5. Verfahren nach Anspruch 4, wobei der Schritt eines Verarbeitens (418a) von Benutzerattribut-Informationen fernes Folgendes umfasst: Priorisieren der Benutzer basierend auf den extrahierten Eignungsindikatoren.

6. Verfahren nach einem der Ansprüche 1 bis 5, wobei die Benutzerattribut-Informationen mindestens eines von Folgenden umfassen:
Informationen über medizinische Qualifikation;
Informationen über Erfahrung mit Wiederbelebung;
Informationen über medizinische oder kardiopulmonale Wiederbelebungsausbildung; und
Informationen über körperliche Verfassung oder Fähigkeiten.

7. Verfahren nach einem der vorherigen Ansprüche, ferner umfassend den folgenden Schritt:
Erfassen (416) von Benutzerattribut-Informationen von der Sensorkomponente einer identifizierten tragbaren Rechenvorrichtung,
wobei die Sensorkomponente optional mindestens eines von Folgenden umfasst: einen Sensor zum Überwachen der Vitalzeichen des Benutzers, der mit dem identifizierten tragbaren Rechenvorrichtung assoziiert ist; und einen Sensor, der angepasst ist, um einen Indikator in Form einer Antwort des Benutzers, der mit dem identifizierten tragbaren Rechenvorrichtung assoziiert ist, auf eine oder mehrere dem Benutzer gestellte Fragen zu erfassen.

8. Verfahren nach einem der vorherigen Ansprüche, wobei der Schritt eines Übermittelns (430) von Anleitungen zur Koordination der kardiopulmonalen Wiederbelebung Folgendes umfasst:
Übermitteln erster Anleitungen zum Ausführen der kardiopulmonalen Wiederbelebung über die Kommunikationsverbindung an eine erste tragbare Rechenvorrichtung, wobei der mit der ersten tragbaren Rechenvorrichtung assoziierte Benutzer ein primärer Benutzer ist; und
Übermitteln von zweiten Anleitungen zum Ausführen der kardiopulmonalen Wiederbelebung über die Kommunikationsverbindung an eine oder mehrere tragbare Computervorrichtungen, die nicht die erste tragbare Computervorrichtung sind, wobei der jeweilige Benutzer oder die jeweiligen Benutzer, die mit der einen oder den mehreren tragbaren Computervorrichtungen assoziiert sind, ein Assistenzbenutzer oder Assistenzbenutzer für die Unterstützung des Hauptbenutzers sind.

9. Verfahren nach einem der vorherigen Ansprüche, ferner umfassend:
die tragbare Koordinationsvorrichtung (100) empfängt Sensorfunktion-Antwortinformationen von einer identifizierten tragbaren Rechenvorrichtung (300, 400), um die Annahme einer Sensorfunktion anzuzeigen, die der Sensorkomponente der identifizierten Vorrichtung zugewiesen ist;
Verarbeiten der Informationen über die Sensorfunktion; und
Übermitteln einer angepassten Anleitung zum Koordinieren der kardiopulmonalen Wiederbelebung von der tragbaren Koordinationsvorrichtung (100) an mindestens eine der identifizierten Vorrichtungen (300, 400) über die Kommunikationsverbindung als Reaktion auf die verarbeiteten Informationen zur Sensorfunktion und die erfassten Daten.

10. Verfahren nach einem der vorherigen Ansprüche, ferner umfassend:
die tragbare Koordinationsvorrichtung (100), empfängt Rollenantwort-Informationen von einer identifizierten tragbaren Computervorrichtung (300, 400), um die Annahme einer zugewiesenen kardiopulmonalen Wiederbelebungsrolle als Reaktion auf die Anleitung zum Koordinieren der kardiopulmonalen Wiederbelebung, die an die identifizierte Vorrichtung übermittelt wurde, anzugeben;
Verarbeiten der Rollenantwort-Informationen; und
Übermitteln einer angepassten Anleitung zum Koordinieren der kardiopulmonalen Wiederbelebung von der tragbaren Koordinationsvorrichtung (100) an mindestens eine der identifizierten Vorrichtungen (300, 400) über die Kommunikationsverbindung als Reaktion auf verarbeitete Rollenantwort-Informationen und die erfassten Daten.

11. Verfahren nach einem der vorherigen Ansprüche, wobei jede der Vielzahl von tragbaren Rechenvorrichtungen (100, 300, 400) mindestens eines von Folgenden umfasst:
ein Smartphone;
eine Smartwatch;
einen Tablet-Computer;
automatischen externen Defibrillator; und
eine am Kopf zu befestigende Rechenvorrichtung mit einem Anzeigemodul, das angeordnet ist, um von einem Träger der am Kopf zu befestigenden Rechenvorrichtung betrachtet werden kann, wenn er die Vorrichtung trägt.

12. Verfahren nach einem der vorherigen Ansprüche, wobei mindestens eine der identifizierten tragbaren Rechenvorrichtungen (300, 400) einen Standortsensor zum Erfassen des Standorts der tragbaren Rechenvorrichtung umfasst, und wobei das Verfahren ferner Folgendes umfasst:
Senden (404) eines Notsignals an einen entfernten Server, wobei das Notsignal Standortinformationen beinhaltet, die den erfassten Standort der tragbaren Rechenvorrichtung angeben.

13. Computerprogrammprodukt, umfassend ein computerlesbares Medium, das einen Computerprogrammcode zum Implementieren des Verfahrens einem der Ansprüche 1 bis 12 verkörpert, wenn er auf einem Prozessor (110) einer tragbaren Koordinationsvorrichtung (100) ausgeführt wird, die mit einem ersten Benutzer assoziiert ist.

14. Koordinierungssystem für kardiopulmonale Wiederbelebung, umfassend das Computerprogrammprodukt nach Anspruch 13, eine tragbare Koordinationsvorrichtung (100), die mit einem ersten Benutzer assoziiert ist, und eine Vielzahl von tragbaren Rechenvorrichtungen (300, 400), die sich in einem Bereich um die tragbare Koordinationsvorrichtung befinden, wobei der Bereich einen zu behandelnden Patienten (10) umfasst, wobei jede der mehreren tragbaren Rechenvorrichtungen mit einem jeweiligen Benutzer (30, 40) assoziiert ist und eine Kommunikationsschnittstelle umfasst, die angepasst ist, um Informationen über eine Kommunikationsverbindung zu übermitteln, und eine Sensorkomponente, die angepasst ist, um Daten des Benutzers (30, 40) der tragbaren Rechenvorrichtung, des Patienten (10) und/oder des Bereichs um die tragbare Koordinationsvorrichtung (100) zu erfassen.

## Revendications

1. Procédé de coordination de réanimation cardiorespiratoire (401) entre:
un dispositif coordinateur portable (100) associé à un premier utilisateur; et une pluralité de dispositifs informatiques portables (300, 400), le dispositif coordinateur portable comprenant une interface de communication agencée pour communiquer des informations via une liaison de communication, le procédé comprenant:
identifiant (406) une pluralité de dispositifs informatiques portables (300, 400) situés dans une zone autour du dispositif coordinateur portable (100), ladite zone comprenant un patient (10) à traiter, chacun de la pluralité de dispositifs informatiques portables étant associé avec un utilisateur respectif (30, 40) et comprenant une interface de communication, agencée pour communiquer des informations via la liaison de communication, et un composant capteur agencé pour acquérir des données de l'utilisateur (30, 40) dudit dispositif informatique portable, le patient (10) et/ou la zone autour du dispositif coordinateur portable (100);
traitement (418a) d'informations d'attributs d'utilisateur relatives aux utilisateurs associés aux dispositifs informatiques portables identifiés de manière à déterminer un indicateur d'adéquation respectif pour chacun des utilisateurs associés aux dispositifs informatiques portables identifiés, ledit indicateur d'adéquation respectif étant indicatif de l'adéquation de l'utilisateur pour effectuer une réanimation cardiopulmonaire sur le patient;
le traitement (418b) d'informations d'attribut de dispositif concernant les dispositifs informatiques portables identifiés, dans lequel le traitement (418b) d'informations d'attribut de dispositif comprend, pour chaque fonctionnalité de capteur respective d'une pluralité de fonctionnalités de capteur, l'extraction d'un indicateur de fonctionnalité respectif (420b) du capteur composant des dispositifs informatiques portables identifiés à partir desdites informations d'attribut de dispositif, ledit indicateur de fonctionnalité respectif étant indicatif de l'adéquation du composant de capteur du dispositif informatique portable identifié pour exécuter ladite fonctionnalité de capteur respective;
pour chaque fonctionnalité de capteur de la pluralité de fonctionnalités de capteur, le dispositif coordinateur portable affectant (421) une fonctionnalité de capteur respective au composant de capteur d'au moins l'un des dispositifs informatiques portables identifiés sur la base des indicateurs d'adéquation et des indicateurs de fonctionnalité;
le dispositif coordinateur portable attribuant (422) un rôle de réanimation cardiopulmonaire à au moins un des utilisateurs associés aux dispositifs informatiques portables identifiés sur la base des indicateurs d'adéquation et des indicateurs de fonctionnalité ci-dessus ;
communiquer (428) des données acquises par au moins l'un des composants de capteur auxquels est attribuée une fonctionnalité de capteur depuis le ou les dispositifs identifiés respectifs vers le dispositif coordinateur portable via la liaison de communication, les données concernant au moins l'un parmi: un signe vital d'un utilisateur associé au dispositif identifié respectif;
un signe vital du patient; et un danger dans la zone autour du dispositif coordinateur portable (100); et communiquer (430) un guidage de coordination de réanimation cardiorespiratoire spécifique du dispositif coordinateur portable à au moins l'un des dispositifs identifiés via la liaison de communication en réponse à l'au moins un rôle de réanimation cardiopulmonaire attribué et aux données acquises.

2. Procédé selon la revendication 1, dans lequel l'étape de traitement (418b) des informations d'attribut de dispositif comprend en outre: la hiérarchisation, pour chaque fonctionnalité de capteur respective, des composants de capteur des dispositifs informatiques portables identifiés sur la base de l'indicateur de fonctionnalité extrait respectif.

3. Procédé selon l'une quelconque des revendications 1 à 2, dans lequel lesdites informations d'attribut de dispositif comprennent au moins l'un parmi:
des composants de capteur disponibles sur le dispositif informatique portable;
spécifications de la batterie;
informations sur le numéro de modèle; et
normes de communication sans fil prises en charge.

4. Procédé selon l'une quelconque des revendications 1 à 3, dans lequel l'étape de traitement des informations d'attribut utilisateur comprend: accéder (416) à une base de données d'informations d'attribut d'utilisateur et extraire des indicateurs d'adéquation (420a) pour chacun des utilisateurs associés aux dispositifs informatiques portables identifiés à partir desdites informations d'attribut d'utilisateur.

5. Procédé selon la revendication 4, dans lequel l'étape de traitement (418a) des informations d'attribut d'utilisateur comprend en outre: la hiérarchisation des utilisateurs sur la base des indicateurs d'adéquation extraits.

6. Procédé selon l'une quelconque des revendications 1 à 5, dans lequel lesdites informations d'attribut d'utilisateur comprennent au moins l'un parmi:
informations sur les qualifications médicales;
informations sur l'expérience de réanimation;
informations sur la formation en réanimation médicale ou cardiopulmonaire; et
information sur la condition physique ou la capacité.

7. Procédé selon l'une quelconque des revendications précédentes, comprenant en outre l'étape consistant à:
capturer (416) des informations d'attribut d'utilisateur à partir du composant capteur d'un dispositif informatique portable identifié, dans lequel ledit composant de capteur comprend facultativement au moins l'un de: un capteur pour surveiller les signes vitaux de l'utilisateur associé au dispositif informatique portable identifié; et un capteur adapté pour capturer un indicateur sous la forme d'une réponse de l'utilisateur associé au dispositif informatique portable identifié à une ou plusieurs questions présentées à l'utilisateur.

8. Procédé selon l'une quelconque des revendications précédentes, dans lequel l'étape de communication (430) d'un guidage de coordination de réanimation cardiopulmonaire comprend:
communiquer des premières instructions pour effectuer une réanimation cardiopulmonaire via la liaison de communication à un premier dispositif informatique portable, l'utilisateur associé au premier dispositif informatique portable étant un utilisateur principal; et
communiquer des secondes instructions pour effectuer une réanimation cardiorespiratoire via la liaison de communication à un ou plusieurs dispositifs informatiques portables autres que le premier dispositif informatique portable, l'utilisateur ou les utilisateurs respectifs associés auxdits un ou plusieurs dispositifs informatiques portables étant un ou des utilisateurs assistant(s) pour assister l'utilisateur principal.

9. Procédé selon l'une quelconque des revendications précédentes, comprenant en outre:
le dispositif coordinateur portable (100) recevant des informations de réponse de fonctionnalité de capteur d'un dispositif informatique portable identifié (300, 400) pour indiquer l'acceptation d'une fonctionnalité de capteur attribuée au composant capteur dudit dispositif identifié;
traiter lesdites informations de réponse de fonctionnalité de capteur; et
communiquer un guidage de coordination de réanimation cardiorespiratoire ajusté du dispositif coordinateur portable (100) à au moins l'un des dispositifs identifiés (300, 400) via la liaison de communication en réponse aux informations de réponse de fonctionnalité de capteur traitées et aux données acquises.

10. Procédé selon l'une quelconque des revendications précédentes, comprenant en outre:
le dispositif coordinateur portable (100) recevant des informations de réponse de rôle d'un dispositif informatique portable identifié (300, 400) pour indiquer l'acceptation d'un rôle de réanimation cardiopulmonaire attribué en réponse au guidage de coordination de réanimation cardiopulmonaire communiqué audit dispositif identifié;
traiter lesdites informations de réponse de rôle; et
communiquer un guidage de coordination de réanimation cardiorespiratoire ajusté du dispositif coordinateur portable (100) à au moins l'un des dispositifs identifiés (300, 400) via la liaison de communication en réponse aux informations de réponse de rôle traitées et aux données acquises.

11. Procédé selon l'une quelconque des revendications précédentes, dans lequel chacun de la pluralité de dispositifs informatiques portables (100, 300, 400) comprend au moins l'un parmi:
un téléphone intelligent;
une montre connectée;
une tablette informatique;
défibrillateur externe automatique; et
un visiocasque ayant un module d'affichage agencé pour être vu par un porteur du visiocasque lorsqu'il porte le dispositif.

12. Procédé selon l'une quelconque des revendications précédentes, dans lequel au moins l'un des dispositifs informatiques portables identifiés (300, 400) comprend un capteur d'emplacement pour détecter l'emplacement du dispositif informatique portable, et dans lequel le procédé comprend en outre:
envoyer (404) un signal de détresse à un serveur distant, ledit signal de détresse comprenant des informations de localisation indiquant la localisation détectée du dispositif informatique portable.

13. Produit programme d'ordinateur comprenant un support lisible par ordinateur incarnant un code de programme d'ordinateur pour mettre en œuvre le procédé de l'une quelconque des revendications 1 à 12 lorsqu'il est exécuté sur un processeur (110) d'un dispositif coordinateur portable (100) associé à un premier utilisateur.

14. Système de coordination de réanimation cardiopulmonaire comprenant le produit de programme informatique selon la revendication 13, un dispositif coordinateur portable (100) associé à un premier utilisateur, et une pluralité de dispositifs informatiques portables (300, 400) situés dans une zone autour du dispositif coordinateur portable, ladite zone comprenant un patient (10) à traiter, chacun de la pluralité de dispositifs informatiques portables étant associé à un utilisateur respectif (30, 40) et comprenant une interface de communication agencée pour communiquer des informations via une liaison de communication, et un composant capteur agencé pour acquérir des données de l'utilisateur (30, 40) dudit dispositif informatique portable, du patient (10) et/ou de la zone autour du dispositif coordinateur portable (100).
